# EUROPÄISCHE PATENTSCHRIFT

(11) **EP 3 496 766 B1**
(45) Veröffentlichungstag und Bekanntmachung des Hinweises auf die Patenterteilung: **09.09.2020**
(21) Anmeldenummer: 17764758.3
(22) Anmeldetag: 01.08.2017
(51) Int. Cl.: A61L 2/26, B67B 1/03, B65B 55/02

(54) **VERFAHREN UND BEHÄLTER ZUM TRANSPORT UND ZUR ÜBERGABE VON STERILEM SCHÜTTFÄHIGEM GUT IN EINEN ISOLATOR**
METHOD AND CONTAINER FOR CONVEYING AND TRANSFERRING STERILE, FREE-FLOWABLE PRODUCTS INTO AN ISOLATOR
PROCÉDÉ ET RÉCIPIENT DE TRANSPORT ET DE TRANSFERT DE PRODUIT COULANT STÉRILE DANS UN ISOLATEUR

(30) Priorität: 09.08.2016 DE 102016009678
(43) Veröffentlichungstag der Anmeldung: 19.06.2019
(60) Teilanmeldung: 19000327.7
(73) Patentinhaber: Atec Pharmatechnik Gmbh, 24966 Sörup (DE)
(72) Erfinder: MUMM, Hans-Werner, 24966 Sörup (DE)
(74) Vertreter: Weidner Stern Jeschke
(86) Internationale Anmeldenummer: PCT/EP2017/000931
(87) Internationale Veröffentlichungsnummer: WO 2018/028821

(56) Entgegenhaltungen:
- EP-A1- 1 510 227
- EP-A1- 2 823 828
- WO-A1-00/74735
- US-A- 5 447 699
- US-A1- 2012 153 610
- US-B1- 6 308 749

## Beschreibung

Die Erfindung betrifft ein Verfahren zur Übergabe von sterilem schüttfähigem Gut aus einem Behälter mit einem schlauchartigen flexiblen Beutelteil und einem dicht mit einem offenen Ende des Beutelteils verbundenen behälterseitigen Teil eines Rapid-Transfer-Port-Systems in einen Isolator gemäß dem Oberbegriff des Anspruchs 1. Bei dem sterilen Gut handelt es sich bevorzugt um gereinigte und sterilisierte kleine Gegenstände für pharmazeutische Zwecke, wie Verschlussstopfen oder -kappen von Vials und Infusionsflaschen oder Teile von Spritzen.

In der pharmazeutischen Industrie werden zum Verschließen von Vials und Infusionsflaschen Verschlussstopfen aus Gummi oder Kunststoff benötigt. Diese Verschlussstopfen müssen gewaschen, sterilisiert und wieder getrocknet werden, bevor sie einer Maschine zum Verschließen der Vials oder Infusionsflaschen zugeführt werden können. Die Reinigung und Sterilisation der Verschlussstopfen oder von ähnlichen kleinen Gegenständen, wie Verschlusskappen, Spritzenteilen oder dergleichen, kann dabei in einer Behandlungsvorrichtung erfolgen, wie sie zum Beispiel in der EP 2 823 828 A1 des Anmelders beschrieben ist. Diese Behandlungsvorrichtung nimmt einen Behandlungs- und Transport-Behälter auf, in den die kleinen Gegenstände als Schüttgut eingefüllt und dann im Behälterinneren mit Wasser, Heißdampf, Trocknungsluft und ggf. anderen Behandlungsmedien beaufschlagt werden. Nach der Behandlung wird das Gut aus dem Behälter in den Isolator übergeben und dort weiterverarbeitet.

Um es zu ermöglichen, das sterile Gut ohne Kontakt mit einer unsterilen Umgebung aus dem sterilen Inneren des Behälters ins sterile Innere des Isolators zu überführen, wird ein so genanntes Rapid-Transfer-Port(RTP)-System verwendet. Das Rapid-Transfer-Port-System umfasst einen an der Befüll- und Entleer-Öffnung des Behälters angebrachten behälterseitigen Teil, der meist als Beta-Teil bezeichnet wird, und einen komplementären, am Isolator angebrachten isolatorseitigen Teil, der meist als Alpha-Teil bezeichnet wird. Vor der Übergabe des Gutes wird der Behälter mit dem behälterseitigen Teil am isolatorseitigen Teil angedockt bzw. mechanisch mit diesem gekuppelt.

Wenn einerseits vorgereinigtes Gut in den Behälter gefüllt wird und andererseits nur kleinere Mengen des Gutes benötigt werden bzw. deren Weiterverarbeitung nur langsam erfolgt, werden statt der in der EP 2 823 828 A1 beschriebenen Behandlungsbehälter aus Edelstahl zum Transport und zur Übergabe in den Isolator manchmal auch Einweg-Behälter aus Kunststoff verwendet, die es auch erlauben, die Reinigung des Gutes von Dritten vornehmen zu lassen. Zur Sterilisierung des Gutes können die Einwegbehälter ggf. beim Transport zum Isolator einer Strahlensterilisation unterzogen oder zur Dampfsterilisation durch einen Autoklav hindurch bewegt werden.

Derartige Kunststoff-Einweg-Behälter werden zum Beispiel von den Firmen Castus, Sartorius oder Getinge La Calhene angeboten und sind auf deren Webseiten http://castus.pro/en/products/beta /item/33-beta-single-use-en.html, https://www.sartorius.de/de/ produkte/bioprozess/aseptischetransfersysteme/biosafe-aseptische-single-use-bag/ bzw. http://www.getinge.com/life-science/products-within/isolation-containmentequipment/dpte-rapid-transfer-port-system/dpte-betabeg/ dargestellt.

Die Kunststoff-Einweg-Behälter bestehen gewöhnlich aus einem schlauchartigen flexiblen Beutelteil, der ein geschlossenes Ende und ein offenes Ende aufweist, in das der behälterseitige Teil des Rapid-Transfer-Port-Systems eingesetzt und dichtend mit dem Beutelteil verbunden ist. Der Beutelteil besteht aus einer durch γ-Strahlung sterilisierbaren Kunststofffolie, die mit einem Andockring des behälterseitigen Teils des Rapid-Transfer-Port-Systems gasdicht verschweißt ist. Der Andockring ist mit einer Ringdichtung ausgestattet und umgibt eine Behälteröffnung, die durch einen abnehmbaren Behälterdeckel dicht verschlossen werden kann. Der isolatorseitige Teil des Rapid-Transfer-Port(RTP)-Systems weist einen komplementären, ebenfalls mit einer Ringdichtung ausgestatteten Andockring auf, der eine Portöffnung umgibt. Die Portöffnung ist durch eine Porttür verschlossen, die sich nur nach dem Andocken eines Behälters öffnen lässt. Beim Andocken des Behälters werden zum einen die beiden Andockringe und zum anderen der Behälterdeckel und die Porttür mechanisch miteinander verriegelt, zum Beispiel durch Drehen des Andockrings des behälterseitigen Teils des RTP-Systems. Beim Verriegeln werden gegenüberliegende unsterile Oberflächen des Behälterdeckels und der Porttür gegeneinander angepresst und dadurch unzugänglich zwischen der Porttür und dem Behälterdeckel eingeschlossen. Anschließend werden die Porttür und der Behälterdeckel im verriegelten Zustand gemeinsam in eine Offenstellung im Inneren des Isolators bewegt, um sowohl die Behälteröffnung und die Portöffnung zur Übergabe des Gutes freizugeben. Nach der Übergabe des Gutes werden die Porttür und der Behälterdeckel wieder in eine Schließstellung zurückbewegt, um zuerst die Behälteröffnung und die Portöffnung zu schließen, dann die beiden Andockringe sowie den Behälterdeckel und die Porttür zu entriegeln und zuletzt den Behälter durch Trennen der beiden Teile des Rapid-Transfer-Port-Systems vom Isolator abzudocken und weg zu bewegen.

Bei der Übergabe des Gutes in den Isolator muss eine ungewollte Kontamination des Isolators verhindert werden. Wenn nach dem Andocken einerseits die beiden Andockringe sowie andererseits die Porttür und der Behälterdeckel miteinander verriegelt sind, liegen ihre gegenüberliegenden unsterilen Oberflächen zwar dicht gegeneinander an und bedecken sich gegenseitig nahezu vollständig. Ausnahmen bilden jedoch zwei schmale ringförmige Flächen an einem inneren Rand der gegeneinander anliegenden unsterilen Oberflächen der Andockringe bzw. von deren Ringdichtungen sowie an einem äußeren Rand der gegeneinander anliegenden unsterilen Oberflächen der Porttür und des Behälterdeckels. Diese Flächen werden auch "Ring of Concern" genannt, weil sie im Hinblick auf die Sterilität Bedenken verursachen (siehe http://www.78stepshealth.us/isolation-technology/ring-of-concern.html). Wenn das Gut nach dem Öffnen der Porttür und des Behälterdeckels aus dem Behälter in den Isolator rutscht, können einige der kleinen Gegenstände mit dem "Ring of Concern" am inneren Rand der Andockringe in Kontakt treten. In diesem Fall ist es denkbar, dass mikrobielle Verunreinigungen oder Endotoxine von diesem "Ring of Concern" auf die Gegenstände übertragen werden und eine Kontamination derselben verursachen.

Um dies zu verhindern, sind die zuvor genannten, von der Firma Getinge La Calhene angebotenen Einweg-Behälter mit einem an beiden Enden offenen Folienschlauchabschnitt versehen, dessen eines Ende wie der flexible Beutelteil mit dem Andockring verschweißt ist. Dieser Folienschlauchabschnitt kann nach dem Andocken des Behälters und dem Öffnen der Porttür und des Behälterdeckels durch die Behälteröffnung und die Portöffnung hindurch aus dem Behälter heraus in den Isolator hinein gezogen werden, so dass das Gut durch den Folienschlauch ins Innere des Isolators rutschen kann. Dazu muss jedoch ein Bediener eine Hand in einen in den Isolator ragenden Handschuh einführen und im Isolator mit dem Handschuh durch die Portöffnung und die Behälteröffnung fassen, um den Schlauchabschnitt zu ergreifen und herauszuziehen. Dieser in "Disposable Technologies for Aseptic Filling" im Photo 3 für einen ähnlichen Behälter dargestellte Vorgang ist jedoch recht umständlich.

Um die Gefahr einer Kontamination des Isolators zu verringern, ist aus der US 8 950 624 B2 bereits ein Alpha-Teil für einen RTP bekannt, bei dem sich die Porttür ohne die Notwendigkeit eines Handschuhs von außen öffnen lässt. Darüber hinaus offenbart diese Druckschrift auch einen Trichter zur Erleichterung der Zufuhr von schüttfähigem Gut in den Isolator, der sich durch externe Betätigung im Inneren des Isolators vor die Innenseite des Alpha-Teils bewegen lässt, jedoch nicht in die Portöffnung hinein, so dass ein Kontakt des Gutes mit dem "Ring of Concern" an den Andockringen nicht sicher verhindert werden kann.

Die US 2012/153610 A1 offenbart ein Verfahren gemäß dem Oberbegriff des Anspruchs 1, bei dem nach Andocken eines behälterseitigen Teils eines Rapid-Transfer-Port-Systems an einem mit einem Film versehenen komplementären isolatorseitigen Teil des Rapid-Transfer-Port-Systems ein Loch in dem Film hergestellt wird, bevor dann die Porttür Port geöffnet und das Gut in den Isolator übergeben wird, wobei sein Kontakt mit dem "Ring of Concern" durch den Film verhindert werden soll.

Die US 5 447 699 A offenbart einen Einweg-Behälter mit einem schlauchartigen flexiblen Beutelteil und einem Andockring eines behälterseitigen Teils eines Rapid-Transfer-Port-Systems. Der Andockring besitzt eine innere Ringschulter, an der eine abziehbare Folie befestigt ist, die eine Öffnung zwischen dem flexiblen Beutelteil und dem Andockring überspannt.

Die US 6,308,749 B1 offenbart ein Verfahren zur Übergabe von sterilen Produkten zwischen einem Behälter mit einem schlauchartigen flexiblen Beutelteil und einem Isolator gemäß dem Oberbegriff des Anspruchs 1.

Die WO 00/74735 A1 offenbart ein Rapid-Transfer-Port-System zur Entleerung eines starren Behälters in einen Isolator, in dem ein Transferrohr schwenkbar montiert ist. Nach dem Andocken des Behälters und wird das Transferrohr in eine Position bewegt, in der es sich durch den geöffneten Rapid-Transfer-Port erstreckt. Jedoch besitzt das Transferrohr dort denselben Außendurchmesser wie ein am Isolator angedockter Rohrabschnitt eines am Behälter angebrachten Y-Verbinders, so dass sich das Transferrohr nur bis zum Stirnende des Rohrabschnitts erstreckt.

Ausgehend hiervon liegt der Erfindung die Aufgabe zugrunde, ein Verfahren der eingangs genannten Art dahingehend zu verbessern, dass bei der Übergabe des Gutes in den Isolator ein Kontakt einzelner Gegenstände mit dem "Ring of Concern" sicher verhindert werden kann, ohne dass der Bediener mit einem Handschuh in den Isolator greifen muss.

Zur Lösung dieser Aufgabe ist das erfindungsgemäße Verfahren dadurch gekennzeichnet, dass aus dem Inneren des Isolators heraus ein Transferrohr durch den geöffneten Rapid-Transfer-Port teilweise in das Innere des Behälters eingeführt wird, und zwar mindestens so weit, dass sein in Einführrichtung vorderes Ende durch den behälterseitigen Teil des Rapid-Transfer-Port-Systems hindurch in den Behälter ragt.

Der in dieser Patentanmeldung verwendete Begriff "Isolator" soll als ein "steriler Reinraum" verstanden werden und zum Beispiel auch ein "Restricted Area Barrier System = RABS" einschließen. Der Begriff "Übergabe von Gut in den Isolator" bedeutet eine Gutübergabe in einen Reinraum einer höheren Reinraumklasse, z.B. von einem Reinraum der Klasse B in einen Isolator oder RABS der Klasse A.

Anders als zum Herausziehen eines Schlauchabschnitts aus dem Behälter ist für eine solche Transferrohrbewegung kein Eingriff in den Isolator erforderlich, da das Transferrohr stets einem gleichbleibenden Bewegungspfad folgt und daher leicht von außen manipuliert werden kann. Grundsätzlich kann das vordere Ende des Transferrohrs durch eine Schwenkbewegung und/oder eine Linearbewegung des Transferrohrs in den Behälter eingeführt werden. Bevorzugt handelt es sich bei der Bewegung des Transferrohrs um eine kombinierte Schwenk- und Linear-Bewegung, bei der das vordere Ende des Transferrohrs zuerst durch eine Schwenkbewegung desselben in den geöffneten Rapid-Transfer-Port und dann durch eine Linearbewegung in den Behälter eingeführt wird. Beim Entleeren des Behälters rutscht das Gut durch das Transferrohr hindurch in den Isolator, wobei das Transferrohr den "Ring of Concern" überbrückt und einen Kontakt des Gutes mit diesem sicher verhindert.

Damit sich das vordere Ende des Transferrohrs durch den geöffneten Rapid-Transfer-Port in den Behälter einführen lässt, muss sein Innenquerschnitt kleiner als der Öffnungsquerschnitt der Portöffnung und der Behälteröffnung sein. Damit das Gut danach durch seine Schwerkraft aus dem Behälter in den Isolator rutscht, muss das in den Behälter eingeführte Transferrohr außerdem schräg nach unten zum Inneren des Isolators hin geneigt sein.

Wenn das vordere Ende eines Transferrohrs, dessen Querschnitt kleiner als der Öffnungsquerschnitt der Portöffnung und der Behälteröffnung ist, in den Behälter eingeführt wird, hat dies ohne zusätzliche Maßnahmen zur Folge, dass bei der Entleerung des Behälters um das vordere Ende des Transferrohrs herum ein Teil des Guts im Behälter zurückgehalten werden kann. Die Menge des zurückgehaltenen Guts wird dabei umso größer, je kleiner der Neigungswinkel zwischen der Längsmittelachse des geneigten Transferrohrs und der von der Port- und Behälteröffnung aufgespannten Ebene ist. Wenn diese Ebene zur Längsmittelachse des geneigten Transferrohrs senkrecht ist, lässt sich das vordere Ende des Transferrohrs bereits bei einem Transferrohr-Querschnitt von etwa vier Fünftel des Öffnungsquerschnitts der Port- und Behälteröffnung durch diese in den Behälter einführen. Demgegenüber darf der Transferrohr-Querschnitt maximal die Hälfte des Öffnungsquerschnitts der Port- und Behälteröffnung betragen, wenn die von diesen aufgespannte Ebene vertikal ausgerichtet ist und mit der Längsachse des geneigten Transferrohrs einen Winkel von 45 Grad einschließt.

Das Zurückhalten von Gut um die Eintrittsöffnung des in den Behälter eingeführten Transferrohrs herum wird gemäß einer bevorzugten Weiterbildung des erfindungsgemäßen Verfahrens dadurch verhindert, dass das vordere Ende des Transferrohrs in einen hinter der Behälteröffnung gelegenen Behälterabschnitt eingeführt wird, dessen Innenquerschnitt sich von der Behälteröffnung weg in Richtung des geschlossenen Endes des Beutelteils verjüngt. Durch diese Maßnahme rutscht das gesamte Gut durch die Querschnittsverengung in das Transferrohr und durch dieses hindurch in den Isolator, wenn zur Entleerung des Behälters entweder dessen hinteres Ende angehoben oder eine zuvor angebrachte Klemmvorrichtung entfernt wird, die dazu dient, vor der Entleerung einen Hindurchtritt von Gut durch die Querschnittsverengung zu verhindern.

Um zu verhindern, dass beim Entleeren des Behälters Gut an der Eintrittsöffnung des Transferrohrs vorbei in den verjüngten Behälterabschnitt gelangen kann, sieht eine weitere bevorzugte Ausgestaltung der Erfindung vor, dass das Transferrohr bis zum Anschlagen seines vorderen Endes gegen den verjüngten Behälterabschnitt in diesen eingeführt wird. Sofern dieser Behälterabschnitt ganz oder teilweise von dem schlauchartigen flexiblen Beutelteil gebildet wird, kann er dabei zweckmäßig zwischen dem Andockring und dem vorderen Ende des Transferrohrs gespannt werden, so dass seine Begrenzungswand gestrafft wird.

Alternativ kann das Straffen der flexiblen Begrenzungswand des verjüngten Behälterabschnitts auch beim Aufhängen des hinteren Endes des Behälters nach dem Andocken erfolgen, zum Beispiel indem man dem Behälterabschnitt eine an die Aufhängvorrichtung angepasste Geometrie verleiht, die beim Anheben und Aufhängen des hinteren Endes für eine Straffung des Behälterabschnitts sorgt.

Um eine vollständige Entleerung des Behälters sicherzustellen und ein Zurückhalten von Gut im Behälter zu verhindern, ist bei dem Behälter zwischen der Behälteröffnung und dem geschlossenen Ende des Beutelteils ein Behälterabschnitt angeordnet, dessen Innenquerschnitt sich vom Andockring weg in Richtung des geschlossenen Endes bis zu einer im Abstand von der Behälteröffnung gelegenen Querschnittsverengung verjüngt, deren Innenquerschnitt kleiner als der Öffnungsquerschnitt der Behälteröffnung ist und bevorzugt weniger als drei Viertel des Öffnungsquerschnitts der Behälteröffnung beträgt.

Durch diese Maßnahmen kann trotz eines im Vergleich zum Öffnungsquerschnitt des Behälters kleineren Transferrohrquerschnitts das im Behälter befindliche Gut bei dessen Entleerung vollständig in die Eintrittsöffnung des Transferrohrs gelenkt werden.

Wenn der Beutelteil auf der vom Andockring abgewandten Seite der Querschnittsverengung einen Innenquerschnitt aufweist, der sich in Richtung der Querschnittsverengung verjüngt, trägt dies ebenfalls dazu bei, dass beim Entleeren kein Gut im Behälter zurückbleibt.

Wenn eine nach dem Andocken des behälterseitigen Teils des Rapid-Transfer-Port-Systems am isolatorseitigen Teil schräg nach unten weisende Mantellinie des zur Querschnittsverengung hin verjüngten Behälterabschnitts mit einer schräg nach unten weisenden Mantellinie eines hinteren Abschnitts des Beutelteils zwischen der Querschnittsverengung und dem geschlossenen Ende des Behälters fluchtet, kann auch dort kein Gut im Behälter zurückbleiben.

Der verjüngte Behälterabschnitt kann entweder von einem Abschnitt des schlauchartigen Beutelteils gebildet werden, der sich von der Querschnittsverengung aus in Richtung des Andockrings trichterförmig erweitert. In diesem Fall besteht der Behälterabschnitt vorzugsweise aus demselben flexiblen Material wie der Beutelteil, der mit seinem zum Andockring benachbarten erweiterten offenen Ende dichtend gegen den Andockring anliegt und mit diesem verschweißt ist. Geeignete Materialien für den verjüngten Behälterabschnitt sind in diesem Fall Polyethylen hoher Dichte, ein Faservlies aus Polyethylen hoher Dichte, das unter der Marke Tyvek® vertrieben wird, oder mit Ethylenvinylacetat (EVA) beladenes Polyethylen.

Alternativ kann der verjüngte Behälterabschnitt jedoch auch von einem allgemein trichterförmigen Fortsatz des Andockrings gebildet werden, der über die vom Isolator abgewandte Seite des Andockrings übersteht und sich zur Querschnittsverengung hin verjüngt. Der verjüngte Behälterabschnitt besteht in diesem Fall bevorzugt aus demselben Material wie der Andockring, z.B. aus Polyethylen, Polyethylen hoher Dichte oder Polysulfon, so dass er beim Spritzgießen des Andockrings einstückig an diesem angeformt werden kann. Bei dieser Alternative liegt der flexible Beutelteil zweckmäßig an der Querschnittsverengung dichtend gegen den Fortsatz an und ist mit diesem verschweißt.

Grundsätzlich könnte jedoch die Verbindung zwischen dem Fortsatz und dem flexiblen Beutelteil an einer beliebigen Stelle zwischen dem Andockring und der Querschnittsverengung angeordnet sein.

Im Folgenden wird die Erfindung anhand von zwei in der Zeichnung schematisch dargestellten Ausführungsformen der Erfindung näher erläutert.
Fig. 1 zeigt eine Seitenansicht eines Behälters, der bei dem erfindungsgemäßen Verfahren verwendet werden kann;
Fig. 2 zeigt eine Seitenansicht eines weiteren Behälters, der bei dem erfindungsgemäßen Verfahren verwendet werden kann;
Fig. 3 zeigt eine vergrößerte Querschnittsansicht des Ausschnitts III aus Fig. 2;
Fig. 4 zeigt eine Ansicht eines in einer vertikalen Wand eines Isolators angeordneten isolatorseitigen Teils eines Rapid-Transfer-Port-Systems zur Übergabe von sterilem schüttfähigem Gut aus dem Behälter aus Fig. 2 und 3 in den Isolator;
Fig. 5 zeigt eine entsprechende Ansicht wie Fig. 4, jedoch nach dem Andocken des Behälters, und mit einem im Inneren des Isolators angeordneten Transferrohr in einer ersten Endstellung;
Fig. 6 zeigt eine entsprechende Ansicht wie Fig. 5, jedoch nach dem gemeinsamen Öffnen einer Porttür und eines Behälterdeckels;
Fig. 7 zeigt eine entsprechende Ansicht wie Fig. 6, jedoch nach dem Bewegen des Transferrohrs in eine zweite Endstellung;
Fig. 8 zeigt eine entsprechende Ansicht wie Fig. 6, jedoch nach dem Anheben eines vom Isolator abgewandten hinteren Teils des Behälters zum Entleeren desselben;
Fig. 9 zeigt eine Ansicht eines weiteren isolatorseitigen Teils eines Rapid-Transfer-Port-Systems in einer schrägen Wand eines Isolators;
Fig. 10 zeigt eine entsprechende Ansicht wie Fig. 9, jedoch nach dem Andocken des Behälters aus Fig. 1, sowie mit einem in der ersten Endstellung befindlichen Transferrohr;
Fig. 11 zeigt eine entsprechende Ansicht wie Fig. 10, jedoch nach dem gemeinsamen Öffnen der Porttür und des Behälterdeckels sowie nach dem Bewegen des Transferrohrs in die zweite Endstellung;
Fig. 12 zeigt eine entsprechende Ansicht wie Fig. 11, jedoch nach dem Anheben des hinteren Teils des Behälters zum Entleeren desselben;
Fig. 13 zeigt eine vergrößerte Querschnittsansicht der beiden Teile des Rapid-Transfer-Ports nach dem Andocken des Behälters aus Fig. 1.

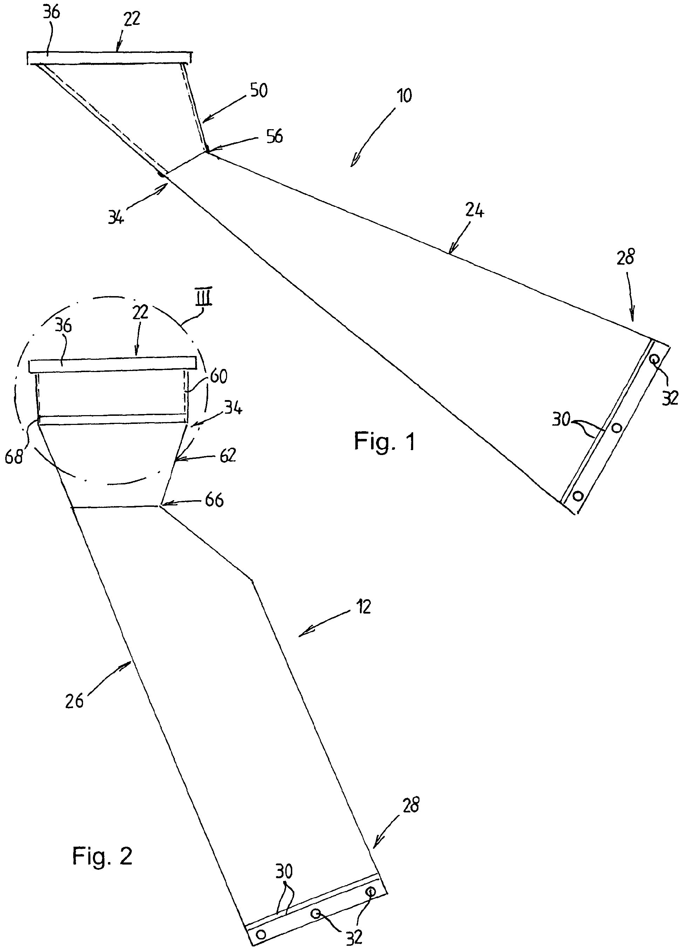

Die in den Figuren 1 und 2 dargestellten Einweg-Behälter 10, 12 dienen zum Transport von zuvor gereinigten und sterilisierten Verschlussstopfen (nicht dargestellt) aus Gummi oder Kunststoff für Vials oder Infusionsflaschen durch einen Reinraum 14 der Reinraumklasse B, zu einem Isolator 16 der Reinraumklasse A, sowie zur Übergabe aus dem Behälter 10, 12 in den Isolator 16, in dem die Verschlussstopfen dann maschinell auf den Vials oder Infusionsflaschen angebracht werden. Die Behälter 10, 12 sind insbesondere für Anwender bestimmt, die kleinere Mengen von Verschlussstopfen benötigen.

Um beim Transport des Behälters 10, 12 zum Isolator 16 und beim Andocken an den Isolator 16 jegliche Kontamination des sterilen Gutes im Behälter 10 oder 12 zu verhindern, sind der Isolator 16 und der Behälter 10, 12 mit einem Rapid-Transfer-Port(RTP)-System 18 ausgestattet. Dabei ist der Isolator 16 mit einem auch als Alpha-Teil bezeichneten isolatorseitigen Teil 20 des RTP-Systems versehen, während der Behälter 10, 12 mit einem als Beta-Teil bezeichneten komplementären behälterseitigen Teil 22 des RTP-Systems 18 versehen ist. Ein geeignetes Rapid-Transfer-Port(RTP)-System wird beispielsweise von der Firma Getinge-LaCalhene unter der Bezeichnung Betabag® angeboten.

Wie am besten in den Figuren 1 bis 3 und 13 dargestellt, bestehen die Einweg-Behälter 10, 12 aus einem schlauchartigen flexiblen Beutelteil 24, 26, sowie dem behälterseitigen Beta-Teil 22 des RTP-Systems 18, der bei beiden Behältern 10, 12 ähnlich ausgebildet ist. Der Beutelteil 24, 26 besteht aus einer Folie aus einem durch γ-Strahlung sterilisierbaren, für Gase und Flüssigkeiten undurchlässigen Kunststoffmaterial, zum Beispiel Polyethylen hoher Dichte oder mit Ethylenvinylacetat (EVA) beladenem Polyethylen. Alternativ kann der Beutelteil 24, 26 auch aus einem dampfdurchlässigen gepressten Vliesmaterial, z.B. Tyvek®, hergestellt werden. Der Beta-Teil 22 ist durch Spritzgießen aus einem durch γ-Strahlung sterilisierbaren Kunststoffmaterial hergestellt, zum Beispiel Polyethylen hoher Dichte oder Polysulfon.

Der Beutelteil 24 des Behälters 10 in Fig. 1 weist einen über die Länge variierenden Querschnitt auf und kann durch gas- und flüssigkeitsdichtes Verschweißen der Ränder von zwei ebenen Zuschnitten hergestellt werden, während der Beutelteil 26 des in Fig. 2 dargestellten Behälters 12 auf einem Teil seiner Länge einen konstanten Querschnitt aufweist und aus einer extrudierten Schlauchfolie hergestellt werden kann. Jeder der Beutelteile 24, 26 weist ein vom Beta-Teil 22 abgewandtes hinteres Ende 28 auf, das durch zwei oder mehr quer verlaufende Schweißnähte 30 gas- und flüssigkeitsdicht verschlossen und hinter den Schweißnähten 30 mit mehreren Ösen 32 versehen ist. In das offene vordere Ende 34 von jedem Beutelteil 24, 26 ist der Beta-Teil 22 des RTP-Systems 18 eingesetzt, wobei die Folie bzw. das Vlies gegen eine äußere Umfangsfläche des Beta-Teils 22 anliegend gas- und flüssigkeitsdicht mit diesem verschweißt ist.

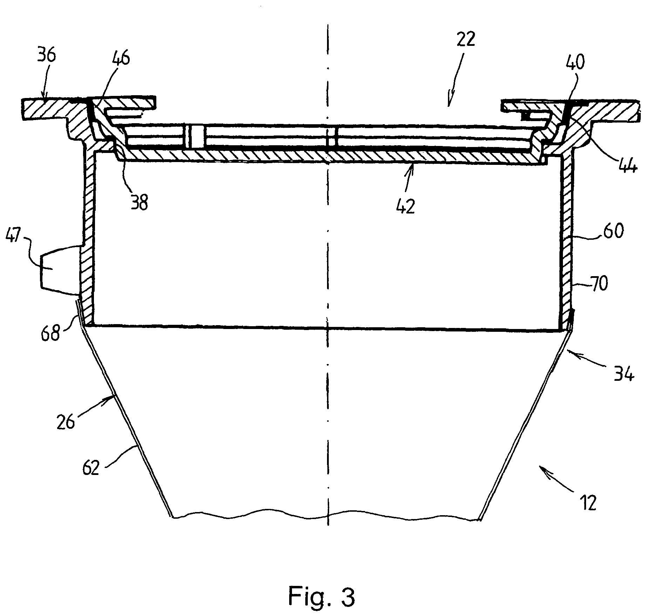

Wie am besten in Fig. 3 dargestellt, besteht der Beta-Teil 22 des RTP-Systems 18 im Wesentlichen aus drei Teilen, nämlich einem Andockring 36, der eine Behälteröffnung 38 umgibt, einer in den Andockring 36 eingesetzten Ringdichtung 40, sowie einem abnehmbaren Behälterdeckel 42, der in geschlossenem Zustand (Fig. 3) die Behälteröffnung 38 gas- und flüssigkeitsdicht verschließt und dazu mit einer äußeren konischen Umfangsfläche 44 dichtend gegen eine komplementäre innere konische Umfangsfläche 46 der Ringdichtung 40 angepresst wird. Der Andockring 36 und der Behälterdeckel 42 weisen komplementäre Eingriffsmittel (nicht sichtbar) auf, mit denen sich der Behälterdeckel 42 zum dichten Verschließen der Behälteröffnung 38 durch Drehen lösbar mit dem Andockring 36 verbinden lässt. Um das Drehen des Behälters 10, 12 zu erleichtern, weist der Beta-Teil 22 radial überstehende Vorsprünge 47 auf, von denen einer in Fig. 3 beispielhaft dargestellt ist.

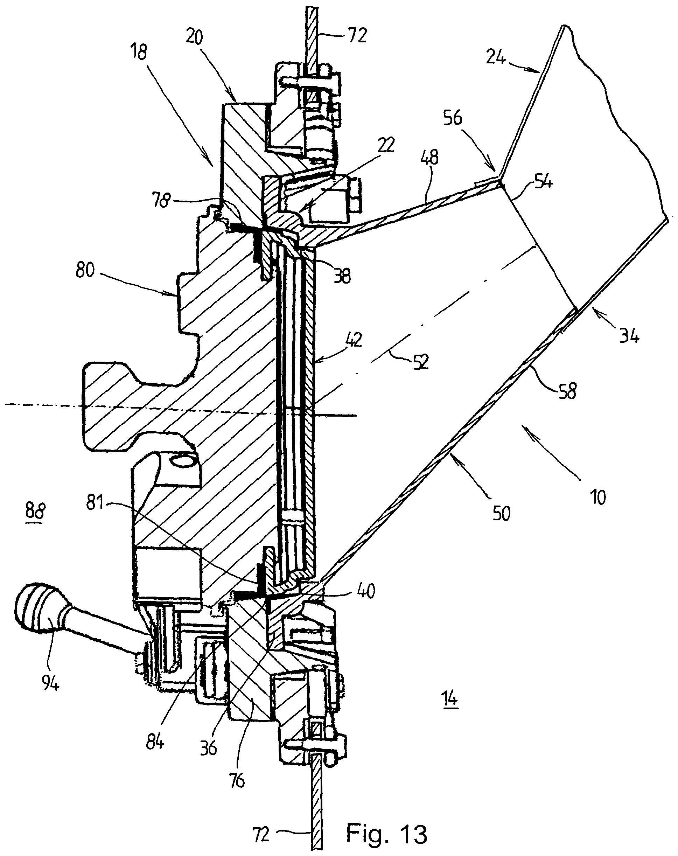

Bei dem Behälter 10 in Fig. 1 und 13 weist der Beta-Teil 22 einen durch Spritzgießen am Andockring 36 angeformten Fortsatz 48 in Form eines hohlen schiefen Kegelstumpfs auf, der einen vom Andockring 36 weg in Richtung des hinteren Endes 28 verjüngten Behälterabschnitt 50 bildet. Der Fortsatz 48 weist an seinem vom Andockring 36 abgewandten hinteren Stirnende einen zur Mittelachse 52 des Kegelstumpfs senkrechten Begrenzungsrand 54 auf, wo die Folie bzw. das Vlies des Beutelteils 24 gegen eine äußere Umfangsfläche 58 des Fortsatzes 48 anliegend gas- und flüssigkeitsdicht mit diesem verschweißt ist. Wie aus Fig. 1 und 13 ersichtlich ist, bildet der Begrenzungsrand 54 eine Querschnittsverengung 56, deren Innenquerschnitt etwa 50 % des Öffnungsquerschnitts der Behälteröffnung 38 beträgt.

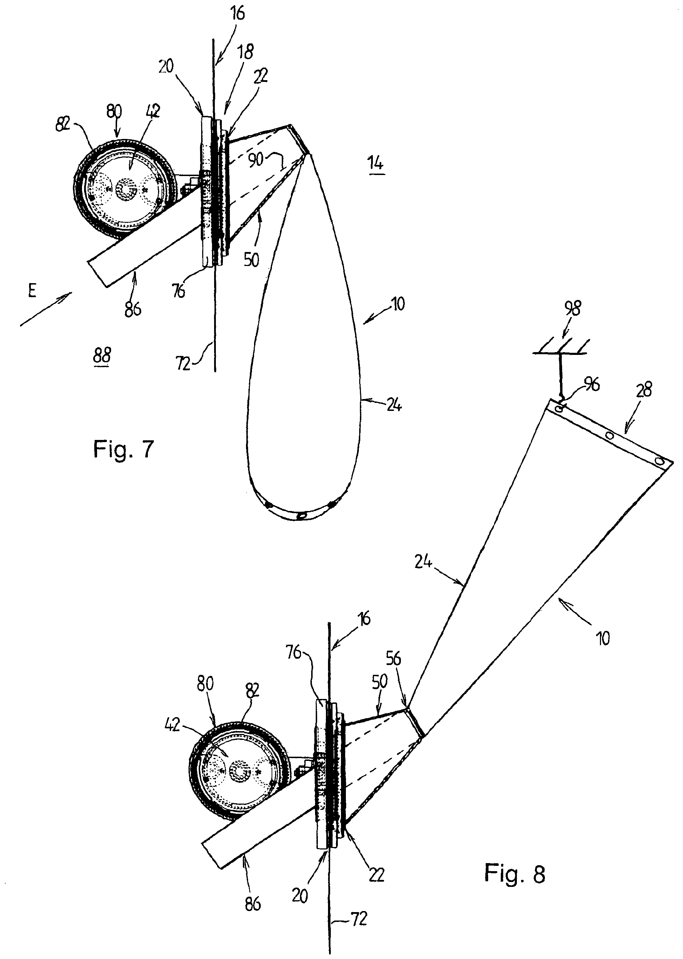

Der Beutelteil 24 des Behälters 10 in Fig. 1 und 13 weist einen vom Begrenzungsrand 54 bis zum hinteren Ende 28 allmählich zunehmenden Querschnitt auf und ist derart mit dem Fortsatz 48 verschweißt, dass beim Entleeren des am Isolator 16 angedockten Behälters 10 eine schräg nach unten gerichtete Mantellinie des kegelstumpfförmigen Fortsatzes 48 mit einer schräg nach unten gerichteten Mantellinie des schlauchförmigen Beutelteils 24 fluchtet, wie in Fig. 8 und 13 dargestellt.

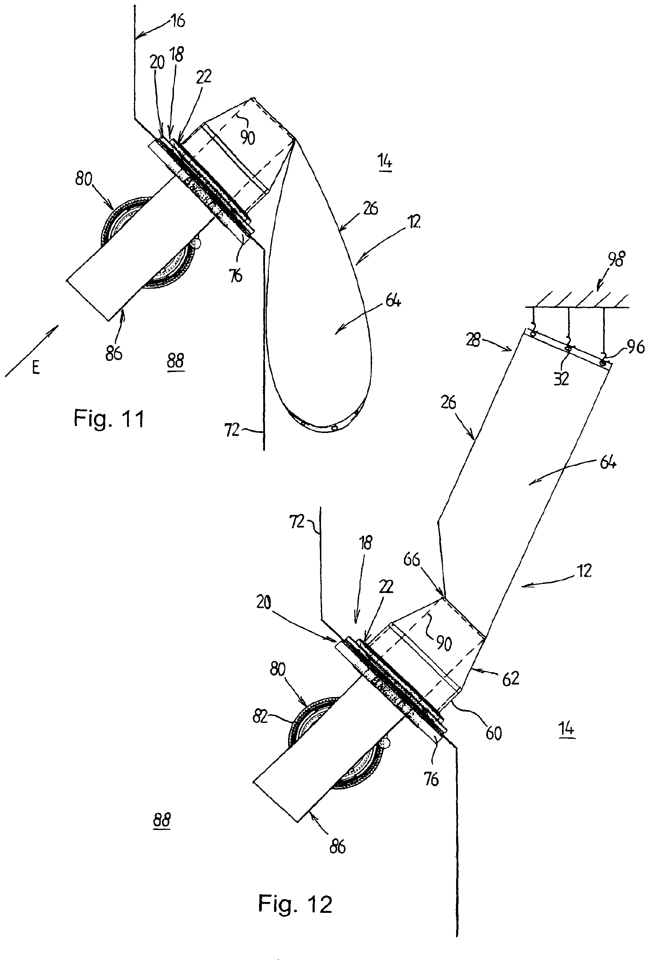

Bei dem Behälter 12 in Fig. 2 und 3 weist der Beta-Teil 22 ebenfalls einen durch Spritzgießen einstückig am Andockring 36 angeformten Fortsatz 60 auf, der ein Stück weit nach hinten über den Andockring 36 übersteht, jedoch hohlzylindrisch ist. Der Beutelteil 26 besteht dort aus zwei einstückig miteinander verbundenen Abschnitten 62, 64, von denen sich der erste 62 vom Andockring 36 bzw. vom Fortsatz 60 weg in Richtung des hinteren Endes 28 bis zu einer Querschnittsverengung 66 trichterförmig verjüngt, wobei er mit seinem vorderen Stirnende 68 dicht gegen eine äußere Umfangsfläche 70 des Fortsatzes 60 anliegend mit diesem verschweißt ist. Der zweite Abschnitt 64 erstreckt sich von der Querschnittsverengung 66 bis zum hinteren Ende 28 des Beutelteils 26, wobei sich sein Innenquerschnitt hinter der Querschnittsverengung 66 zuerst erweitert und dann bis zum hinteren Ende 28 konstant bleibt. Der Innenquerschnitt der Querschnittsverengung 66 ist dort größer als bei dem Behälter 10 in Fig. 1 und beträgt etwa 65 % des Öffnungsquerschnitts der Behälteröffnung 38. Die beiden Abschnitte 62, 64 sind so geformt und miteinander verbunden, dass beim Entleeren des am Isolator 16 angedockten Behälters 12 zwei schräg nach unten gerichtete Mantellinien der beiden Abschnitte 62, 64 miteinander fluchten, wie in Fig. 12 dargestellt.

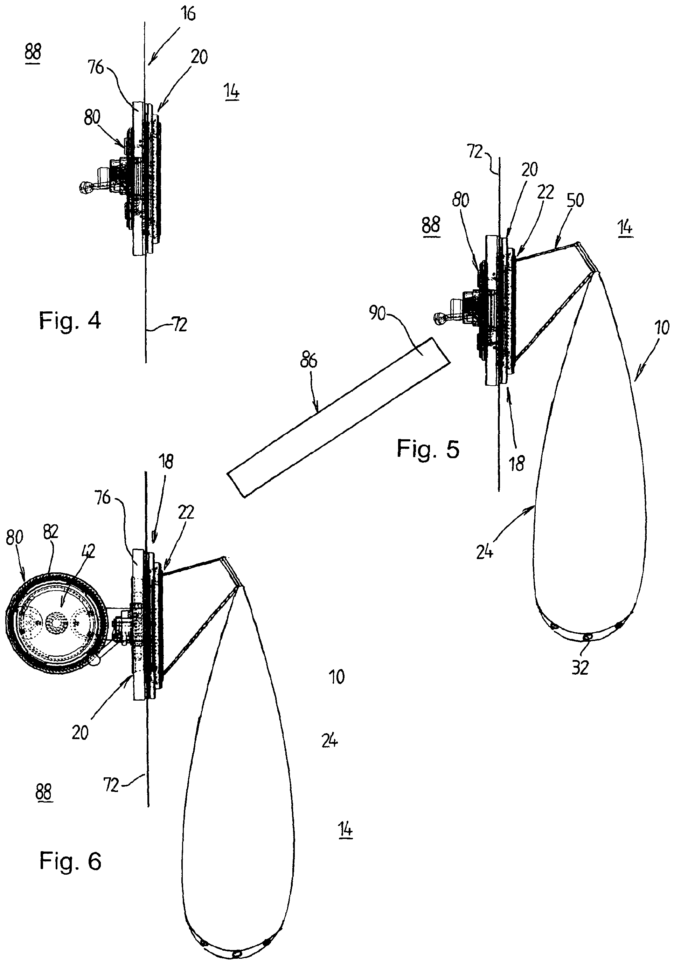

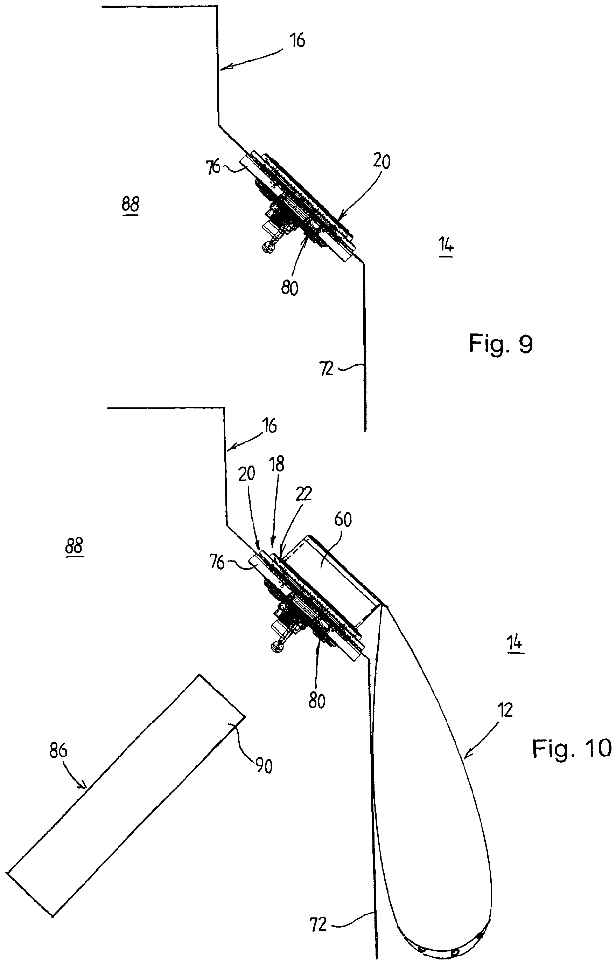

Wie am besten in den Figuren 4 und 9 dargestellt, ist der Alpha-Teil 20 des RTP-Systems 18 dichtend in eine dem Reinraum 14 zugewandte Begrenzungswand 72 des Isolators 16 eingesetzt. Bei dem Isolator 16 in den Figuren 4 bis 8 ist die Wand 72 vertikal ausgerichtet, während sie bei dem Isolator 16 in den Figuren 9 bis 12 unter einem Winkel von 45 Grad zur Vertikalen geneigt ist.

Wie am besten in Fig. 13 dargestellt, umfasst der Alpha-Teil 20 einen in eine Wandöffnung der Begrenzungswand 72 eingesetzten und dichtend mit der Wand 72 verbunden Andockring 76, der eine Portöffnung 78 umgibt, eine Porttür 80 und eine an der Porttür 80 angebrachte Ringdichtung 81. Die Portöffnung 78 ist durch die Porttür 80 dicht verschlossen, die nach dem Andocken des Behälters 10, 12 zur Übergabe des Gutes unter Freigabe der Portöffnung 78 entriegelt und geöffnet werden kann, wie zum Beispiel in Fig. 6 dargestellt.

Die Andockringe 76 und 36 des Alpha-Teils 20 und des Beta-Teils 22 sowie die Porttür 80 und der Behälterdeckel 42 sind jeweils mit paarweise komplementären Kupplungseinrichtungen (nicht sichtbar) versehen, die miteinander in Kupplungseingriff treten, wenn der Beta-Teil 22 beim Andocken am Alpha-Teil 20 in Bezug zu diesem um eine Mittelachse beider Teile 20, 22 gedreht wird. Durch den Kupplungseingriff lassen sich danach die Porttür 80 und der Behälterdeckel 42 nur gemeinsam öffnen und schließen. Die Kupplungseinrichtungen sorgen außerdem dafür, dass die gegenüberliegenden unsterilen Oberflächen der beiden Andockringe 76 und 36 einerseits sowie der Porttür 80 und des Behälterdeckels 42 andererseits miteinander zur Anlage gebracht und dicht gegeneinander angepresst werden.

Abgesehen von zwei "Ring of Concern" genannten schmalen umlaufenden Flächen bedecken sich die gegeneinander anliegenden unsterilen Oberflächen nahezu vollständig, wodurch eine eventuelle Kontamination des Isolators 16 weitestgehend verhindert werden kann. In den Figuren 6 bis 8 sowie 11 und 12 ist der "Ring of Concern" am Rand der gegeneinander anliegenden Flächen der Porttür 80 und des Behälterdeckels 42 mit dem Bezugszeichen 82 bezeichnet, während der "Ring of Concern" am Rand der gegenüberliegenden unsterilen Oberflächen der Andockringe 76 und 36 in Fig. 13 mit dem Bezugszeichen 84 bezeichnet ist.

Um zu verhindern, dass bei der Übergabe des Gutes aus dem Behälter 10, 12 in den Isolator 16 einzelne Verschlusstopfen mit dem "Ring of Concern" 84 Kontakt treten können, ist im Inneren 88 des Isolators 16 ein hohlzylindrisches Transferrohr 86 montiert, mit dem bei geöffneter Porttür 80 das Gut aus dem Behälter 10, 12 über den "Ring of Concern" 84 hinweg durch die Behälteröffnung 38 und die Portöffnung 78 ins Innere 88 des Isolators 16 überführt werden kann. Dazu lässt sich das Transferrohr 86 zwischen einer ersten, in den Figuren 5 bzw. 7 dargestellten Stellung und einer zweiten, in den Figuren 7 bzw. 11 und 12 dargestellten Stellung entlang seiner Längsachse bewegen, um es bei geöffneter Porttür 80 durch die Portöffnung 78 und die Behälteröffnung 38 teilweise in den Behälter 10 bzw. 12 einzuführen.

In der ersten Stellung befindet sich das gesamte Transferrohr 86 außerhalb des Schwenkwegs der Porttür 80 im Inneren 88 des Isolators 16. In der zweiten Stellung ragt das Transferrohr 86 mit seinem in Einführrichtung E vorderen Endabschnitt 90 in den zur Querschnittsverengung 56 bzw. 66 hin verjüngten Abschnitt 50 bzw. 62 des Behälters 10 bzw. 12. Dabei liegt das vordere Stirnende des Transferrohrs 86 an oder ggf. auch in der Nähe der Querschnittsverengung 56 bzw. 66 gegen die Begrenzungswand des Behälterabschnitts 50 bzw. 62 an, so dass kein Gut in den Zwischenraum zwischen dem äußeren Umfang des Transferrohrs 86 und der Begrenzungswand eindringen kann.

Wie am besten durch Vergleich der Figuren 9 und 10 ersichtlich ist, wird beim Einführen des Transferrohrs 86 in den Behälter 12 aus den Figuren 2, 3 und 10 bis 12 nach dem Kontakt des vorderen Stirnendes des Transferrohrs 86 mit der flexiblen Begrenzungswand des Behälterabschnitts 62 die letztere gestreckt oder gestrafft, bis sie schließlich an oder nahe der Querschnittsverengung 66 gegen das vordere Stirnende des Transferrohrs 86 angepresst wird und zwischen diesem und dem vorderen Ende des Beutelteils 26 unter einer gewissen Spannung steht. Auch bei dem Behälter 10 wird das Transferrohr 86 in der zweiten Stellung zweckmäßig mit seinem vorderen Ende leicht gegen die Begrenzungswand des hohlen Fortsatzes 48 angepresst, wie in Fig. 7 und 8 dargestellt.

Obwohl bei den in der Zeichnung dargestellten Ausführungsformen die Bewegung des Transferrohrs 86 eine Linearbewegung ist, kann es sich auch um eine kombinierte Schwenk- und Linearbewegung handeln, zum Beispiel eine Bewegung, bei der das Transferrohr 86 in der Ebene der Zeichnung zuerst aus der ersten Stellung um eine zur Zeichnungsebene senkrechte Schwenkachse geschwenkt und dann entlang seiner Längsachse durch die Portöffnung 78 und die Behälteröffnung 38 in den Behälter 10, 12 eingeführt wird.

Wie durch Vergleich der Figuren 7 und 8 einerseits sowie 11 und 12 andererseits ersichtlich ist, kann der Querschnitt des Transferrohrs 86 und damit auch der Innenquerschnitt der Querschnittsverengung 56 bzw. 66 mit zunehmendem Neigungswinkel zwischen der Längsmittelachse des Transferrohrs 86 und der von der Portöffnung 78 bzw. der Begrenzungswand 72 des Isolators 16 aufgespannten Ebene größer gemacht werden, so dass das Gut aus dem Behälter 10, 12 schneller in den Isolator 16 rutscht. Bei vielen Isolatoren 16 ist jedoch aus Platzgründen der Alpha-Teil 20 in einer vertikalen Begrenzungswand 72 angeordnet.

Im Folgenden wird unter Bezugnahme auf die Figuren 4 bis 8 und 9 bis 12 das Verfahren zum Andocken des Behälters 10, 12 und zur Übergabe des Gutes in den Isolator 16 kurz erläutert.

Zuerst wird der Behälter 10, 12 zum Isolator 16 transportiert und der Beta-Teil 22 in einer vorbestimmten Drehausrichtung an den Alpha-Teil 20 angenähert, so dass sich die komplementären Kupplungseinrichtungen zuerst in Eingriff bringen und dann durch Drehen des Behälters 10, 12 um die Längsmittelachse des Beta-Teils 22 miteinander verriegeln lassen, wie in den Figuren 5 bzw. 10 dargestellt.

Nach dem Andocken ist der Behälter 10, 12 dann so ausgerichtet, dass der Beutelteil 24 bzw. 26 nach unten hängt, wie in den Figuren 5 bzw. 10 dargestellt. Dadurch kann das Eindringen von Gut aus dem Behälter 10, 12 in den verjüngten Behälterabschnitt 50 bzw. 62 verhindert werden.

Das gemeinsame Entriegeln und Öffnen der Porttür 80 und des Behälterdeckels 42 sowie die Bewegung des Transferrohrs 86 aus der ersten in die zweite Stellung kann vom Reinraum 14 aus vorgenommen werden. Zum Entriegeln und Öffnen der Porttür 80 sowie zum Bewegen des Transferrohrs 86 geeignete Einrichtungen sind in der eingangs genannten US 8 950 624 B2 beschrieben und sollen daher hier nicht näher erläutert werden. Sofern der Isolator 16 mit einem in der Nähe der Portöffnung 78 in sein Inneres ragenden Handschuh (nicht dargestellt) und einem innen am Ringflansch angebrachten Entriegelungshebel 94 versehen ist, kann die Porttür 80 auch geöffnet werden, indem ein Bediener eine Hand in den Handschuh einführt, den Entriegelungshebel 94 betätigt und dann die Porttür 80 nach einer Seite hin in Innere 88 des Isolators 16 hinein schwenkt, wie z.B. in Fig. 6 und 11 dargestellt.

Anschließend wird das Transferrohr 86 vom Reinraum her aus der ersten Stellung (Fig. 5 bzw. 10) in die zweite Stellung (Fig. 6 bzw. 11) bewegt, um seinen vorderen Endabschnitt 90 durch die Portöffnung 78 und die Behälteröffnung 38 hindurch in den verjüngten Behälterabschnitt 50 bzw. 62 einzuführen, bis sein vorderes Stirnende unter leichtem Druck von innen her gegen dessen Begrenzungswand anliegt.

Danach kann der schlauchartige flexible Beutelteil 24 des Behälters 10 bzw. der hintere erste Abschnitt 64 des Beutelteils 26 des Behälters 12 zum Entleeren des Behälters 10 bzw. 12 angehoben und sein hinteres Ende 28 mit Hilfe von einer oder mehreren der Ösen 32 an einem oder mehreren Haken 96 einer zu diesem Zweck im Reinraum 14 vorgesehenen Aufhängvorrichtung 98 aufgehängt werden. Die Aufhängvorrichtung 98 ist dabei so an den Behälter 10 bzw. 12 angepasst, dass nach dem Anheben des Beutelteils 24 bzw. des zweiten Abschnitts 64 des Beutelteils 26 die entlang des Beutelteils 24 bzw. der Behälterabschnitte 64 und 62 verlaufende, nach unten weisende Mantellinie gerade und beim Behälter 10 unter einem Winkel von weniger als 50 Grad bzw. beim Behälter 12 unter einem Winkel von weniger als 40 Grad zur Vertikalen geneigt ist.

Alternativ kann der Beutelteil 24 des Behälters 10 bzw. der hintere Abschnitt 64 des Beutelteils 26 des Behälters 12 bereits beim Andocken mit seinem hinteren Ende 28 an der Aufhängvorrichtung 98 aufgehängt werden, sofern er hinter der Querschnittsverengung 56 bzw. 66 mit einer lösbaren oder abnehmbaren Klemmvorrichtung (nicht dargestellt) versehen ist, welche die gegenüberliegenden Begrenzungswände des Beutelteils 24 bzw. des Abschnitts 64 dort über den gesamten Querschnitt gegeneinander anpresst und dadurch vor dem Lösen oder Abnehmen der Klemmvorrichtung jeglichen Eintritt von Gut aus dem Beutelteil 24 bzw. Abschnitt 64 durch die Querschnittsverengung 56 bzw. 66 in den Abschnitt 50 bzw. 62 verhindert. In diesem Fall kann die Klemmvorrichtung zum Entleeren des Behälters 10, 12 gelöst oder abgenommen werden, sobald sich das Transferrohr 86 in der zweiten Stellung befindet.

In beiden Fällen kann das Straffen oder Strecken der flexiblen Begrenzungswand des Behälterabschnitts 62 des Behälters 12 statt durch das Einführen des Transferrohrs 86 auch durch eine geeignete Positionierung und Ausbildung der Aufhängvorrichtung 98 (Fig. 12) erfolgen, die im Zusammenwirken mit dem Behälter 12 bzw. dem Beutelteil 26 beim Aufhängen von dessen hinterem Ende 28 für die Straffung oder Streckung des flexiblen Folien- oder Vliesmaterials des Behälterabschnitts 62 sorgt.

Nach dem Entleeren des Behälters 10, 12 wird zuerst das Transferrohr 86 in die erste Stellung zurück bewegt, bevor die Porttür 80 mit dem Behälterdeckel 42 geschlossen wird. Dann werden die Porttür 80 im Andockring 76 des Alpha-Teils 20 und der Behälterdeckel 42 im Andockring 36 des Beta-Teils 22 verriegelt. Anschließend wird der Beta-Teil 20 des Behälters 10, 12 zum Ausrücken der komplementären Kupplungseinrichtungen der RTP-Systems 18 gedreht und dann vom Alpha-Teil 20 abgenommen, um ihn erneut mit Verschlussstopfen zu befüllen.

Selbstverständlich ist es auch möglich, den Behälter 10 in Fig. 1 statt mit dem mit dem Andockring 36 integralen Fortsatz 48 mit einem verjüngten Behälterabschnitt 50 aus einem flexiblen Folien- oder Vliesmaterial zu versehen und den verjüngten Behälterabschnitt 62 des Behälters 12 in Fig. 2 statt aus einem flexiblen Folien- oder Vliesmaterial aus einem zusammen mit dem Andockring 36 durch Spritzgießen geformten hohlen Fortsatz zu versehen bzw. den Fortsatz 60 trichterförmig auszubilden.

## Patentansprüche

1. Verfahren zur Übergabe von sterilem schüttfähigem Gut, insbesondere von gereinigten und sterilisierten kleinen Gegenständen, wie Verschlussstopfen von Infusionsflaschen und Vials, aus einem Behälter (10; 12) mit einem schlauchartigen flexiblen Beutelteil (24; 26) und einem dicht mit einem offenen Ende (34) des Beutelteils (24; 26) verbundenen behälterseitigen Teil (22) eines Rapid-Transfer-Port-Systems (18) in einen Isolator (16), wobei das Gut nach Andocken des behälterseitigen Teils (22) des Rapid-Transfer-Port-Systems (18) an einem komplementären isolatorseitigen Teil (20) des Rapid-Transfer-Port-Systems (18) und nach dem Öffnen des Rapid-Transfer-Ports in den Isolator (16) übergeben wird, **dadurch gekennzeichnet, dass** aus dem Inneren des Isolators (16) heraus ein Transferrohr (86) durch den geöffneten Rapid-Transfer-Port teilweise in das Innere des Behälters (10, 12) eingeführt wird.

2. Verfahren nach Anspruch 1, **dadurch gekennzeichnet, dass** ein in Einführrichtung vorderer Endabschnitt (90) des Transferrohrs (86) in einen Behälterabschnitt (50; 62) eingeführt wird, dessen Innenquerschnitt sich von der Behälteröffnung (38) weg in Richtung eines geschlossenen Endes (28) des Beutelteils (24; 26) verjüngt.

3. Verfahren nach Anspruch 2, **dadurch gekennzeichnet, dass** das Transferrohr (86) bis zum Anschlagen seines in Einführrichtung (E) vorderen Stirnendes gegen eine Begrenzungswand des Behälterabschnitts (50; 62) in den Behälter (10; 12) eingeführt wird.

4. Verfahren nach Anspruch 3, **dadurch gekennzeichnet, dass** der Behälterabschnitt (50; 62) mindestens teilweise von dem schlauchartigen flexiblen Beutelteil (26) gebildet wird, der beim Einführen des Transferrohrs (86) bis zum Anschlagen seines vorderen Stirnendes gegen den Behälterabschnitt (50; 62) mindestens zum Teil gespannt oder gestrafft wird.

5. Verfahren nach einem der Ansprüche 2 bis 4, **dadurch gekennzeichnet, dass** der vordere Endabschnitt (90) des Transferrohrs (86) durch eine Schwenkbewegung oder eine Linearbewegung des Transferrohrs (86) in den geöffneten Rapid-Transfer-Port eingeführt wird und dann durch eine Linearbewegung des Transferrohrs (86) teilweise in den Behälter (10; 12) eingeführt wird.

## Claims

1. A method for transferring sterile pourable goods, in particular cleaned and sterilized small objects, such as stoppers of infusion bottles and vials, from a container (10; 12) having a hose-like flexible bag part (24; 26) and a container-side part (22) of a rapid-transfer-port-system tightly connected with an open end (34) of the bag part (24; 26) into an isolator (16), wherein after the container-side part (22) of the rapid-transfer-port-system (18) has docked with a complementary isolator-side part (20) of the rapid-transfer-port-system (18) and after the rapid-transfer-port has been opened, the goods are transferred into the isolator (16),
**characterized in that** a transfer tube (86) is partially introduced from the interior of the isolator (16) into the interior of the container (10, 12) through the open rapid-transfer-port.

2. The method according to claim 1, **characterized in that** a front end section (90) of the transfer tube (86) in the direction of introduction is introduced into a container section (50; 62), the inner cross-section of which tapers from the container opening (38) in the direction of a closed end (28) of the bag part (24; 26).

3. The method according to claim 2, **characterized in that** the transfer tube (86) is introduced into the container (10; 12) until its front end face in the direction of introduction (E) abuts against a boundary wall of the container section (50; 62).

4. The method according to claim 3, **characterized in that** the container section (50; 62) is formed at least partially by the hose-like flexible bag part (26), which is at least partially tensioned or tautened, when the transfer tube (86) is introduced until its front end face abuts against the container section (50; 62).

5. The method according to one of the claims 2 to 4, **characterized in that** the front end section (90) of the transfer tube (86) is introduced into the opened rapid-transfer-port-system by a pivoting motion or a linear motion of the transfer tube (86) and then partially introduced into the container (10, 12) by a linear motion of the transfer tube (86).

## Revendications

1. Procédé de transfert de produits stériles en vrac, en particulier de petits objets nettoyés et stérilisés, tels que des bouchons de flacons de perfusion et de fioles, à partir d'un récipient (10 ; 12) comportant une partie de sac souple de type tuyau (24 ; 26) et une partie côté récipient (10, 12) d'un système de port de transfert rapide (18) reliée de manière étanche avec une extrémité ouverte (34) de la partie de sac (24 ; 26) dans un isolateur (16), où après l'amarrage de la partie côté récipient (22) du système de port de transfert rapide (18) à une partie côté isolateur complémentaire (20) du système de port de transfert rapide (18) et après l'ouverture du port de transfert rapide, les produits sont transférés dans l'isolateur (16), **caractérisé en ce qu'**un tube de transfert (86) est partiellement introduit depuis l'intérieur de l'isolateur (16) à travers le port de transfert rapide ouvert dans l'intérieur du récipient (10, 12).

2. Procédé selon la revendication 1, **caractérisé en ce qu'**une section d'extrémité avant (90) du tube de transfert (86) dans la direction d'introduction est introduite dans une section de récipient (50 ; 62) dont la section transversale intérieure s'amincit depuis l'ouverture de récipient (38) en direction d'une extrémité fermée (28) de la partie de sac (24 ; 26).

3. Procédé selon la revendication 2, **caractérisé en ce que** le tube de transfert (86) est introduit dans le récipient (10 ; 12) jusqu'à ce que son extrémité frontale avant dans la direction d'introduction (E) bute contre une paroi de délimitation de la section de récipient (50 ; 62).

4. Procédé selon la revendication 3, **caractérisé en ce que** la section de récipient (50 ; 62) est au moins partiellement formée par la partie de sac (26) souple de type tuyau, qui est au moins partiellement tendue ou serrée lors de l'introduction du tube de transfert (86) jusqu'à ce que son extrémité frontale avant bute contre la section de récipient (50 ; 62).

5. Procédé selon l'une des revendications 2 à 4, **caractérisé en ce que** la section d'extrémité avant (90) du tube de transfert (86) est introduite dans le port de transfert rapide ouvert par un mouvement de pivotement ou un mouvement linéaire du tube de transfert (86) puis partiellement introduite dans le récipient (10 ; 12) par un mouvement linéaire du tube de transfert (86).
